# EUROPÄISCHE PATENTSCHRIFT

(11) **EP 3 760 236 B1**
(45) Veröffentlichungstag und Bekanntmachung des Hinweises auf die Patenterteilung: **04.09.2024**
(21) Anmeldenummer: 20184297.8
(22) Anmeldetag: 06.07.2020
(51) Int. Cl.: A61L 2/07, B67C 3/00

(54) **REINIGUNGSVALIDIERUNG IN EINER VORRICHTUNG ZUM ABFÜLLEN VON BEHÄLTERN**
VALIDATION OF CLEANING IN A CONTAINER FILLING DEVICE
VALIDATION DU NETTOYAGE DANS UN DISPOSITIF DE REMPLISSAGE DE RÉCIPIENTS

(30) Priorität: 04.07.2019 DE 102019118114
(43) Veröffentlichungstag der Anmeldung: 06.01.2021
(73) Patentinhaber: KRONES AG, 93073 Neutraubling (DE)
(72) Erfinder: Knott, Josef, 93073 Neutraubling (DE); Mueller, Holger, 93073 Neutraubling (DE); Habersetzer, Florian, 93073 Neutraubling (DE)
(74) Vertreter: Nordmeyer, Philipp Werner

(56) Entgegenhaltungen:
- EP-A1- 0 418 079
- EP-A1- 3 015 418
- WO-A1-2018/104551
- WO-A2-2019/002464
- JP-A- 2000 355 397

## Beschreibung

### Technisches Gebiet

Die vorliegende Erfindung betrifft eine Vorrichtung zum Befüllen eines Behälters mit einem Füllprodukt, vorzugsweise in einer Getränkeabfüllanlage, sowie ein Verfahren zum Reinigen und/oder Sterilisieren einer solchen Vorrichtung.

### Hintergrund der Erfindung

Es sind verschiedene Verfahren zur Reinigung und Sterilisierung von Füllvorrichtungen zum Abfüllen von Behältern mit einem Füllprodukt, etwa Getränken, bekannt. So haben sich beispielsweise das sogenannte CIP-Verfahren ("Cleaning-In-Place") und SIP-Verfahren ("Sterilization-In-Place") etabliert, bei denen die Füllventile zur Reinigung beziehungsweise Sterilisation nicht ausgebaut werden müssen, sondern im eingebauten Zustand mit einem Reinigungsmedium oder Sterilisierungsmedium durchspült beziehungsweise bedämpft werden. Beispielsweise werden die Füllventile, insbesondere die mit dem Füllprodukt in Berührung kommenden Oberflächen, mittels Heißdampf thermisch sterilisiert. Dabei werden die Füllventile mit Heißdampf beaufschlagt und auf diese Weise auf die Sterilisierungstemperatur gebracht. Die daraus resultierende Temperatur der Füllventile wird über eine bestimmte Zeit aufrechterhalten und überwacht, um eine vorgegebene Sterilisationswirkung zu erzielen, wie es beispielsweise aus der DE 10 2010 031 873 A1, DE 10 2013113621 A1, EP 3 015 418 A1, JP2000355397A und EP 0 418 079 A1 bekannt ist.

Zur Reduzierung des Dampfverbrauchs bei gleichzeitiger Aufrechterhaltung des Drucks, zur Ableitung von Kondensat und zur besseren Detektion des Dampfdurchsatzes kann während der thermischen Sterilisation eine Blende mit einer kleinen Öffnung vor den Auslauf des Füllventils geschwenkt und dichtend mit diesem verbunden werden. Durch die kleine Öffnung der Blende hindurch tritt dann ein Dampfstrahl aus, dessen dynamischer Druck mittels eines unterhalb der Blende angeordneten Drucksensors detektiert wird. Die Temperatur wird dabei indirekt durch das Erfassen des vorgelagerten Sattdampfdruckes und des Dampfdurchsatzes beziehungsweise Dampfdruckes im Füllventil über den somit erfassten Differenzdruck pro Füllventil ermittelt. Üblicherweise ist bei Rotationsmaschinen der Drucksensor statisch unterhalb der Höhe der Blende verbaut. Der Druck des aus der Blende austretenden Dampfstrahls wird dabei erfasst, wenn das jeweilige Füllventil über den Drucksensor bewegt wird.

Insbesondere wenn für hygienische Anwendungen das Abfüllen des Füllprodukts in einem Abfüllraum beziehungsweise Reinraum der Füllvorrichtung erfolgt, in dem im Vergleich zur Umgebung eine reduzierte mikrobielle Belastung und/oder ein Überdruck vorliegt und mithin der Sterilitätsgrad gegenüber der Umgebung erhöht ist, ist die Installation zusätzlicher Bauteile und Komponenten, wie etwa einer Blende, einer Hub- und Schwenkvorrichtung für die Blende, eines Drucksensors und dergleichen, aus hygienischer Sicht nachteilig, da die zusätzlich im Reinraum verbauten Bauteile zu einem erhöhten Reinigungsaufwand führen.

Eine weitere Möglichkeit der Temperaturüberwachung besteht nach dem Stand der Technik darin, jedes einzelne Füllventil mit einem Thermoelement zu überwachen, welches in der Regel in dem Füllventil integriert oder an der Blende angeordnet ist. Dies erfordert jedoch ein Thermoelement pro Füllventil beziehungsweise pro Blende sowie eine entsprechend aufwendige Verdrahtung, welche bei Rundläufermaschinen im drehenden Teil verbaut ist.

Eine Temperaturüberwachung der Füllventile während der Sterilisation mittels eines berührungslosen Temperatursensors ist aus der WO 2018/104551 A1 bekannt.

Es ist bei konventionellen Füllsystemen zudem bekannt, die Reinigung mit Hilfe der zur Befüllung der Behälter genutzten Durchflussmesser zu validieren. Wird während der Reinigung am zu validierenden Füllventil ein eingestellter Durchfluss-Schwellwert überschritten, so wird die Reinigung als erfolgreich angesehen.

Allerdings sind inzwischen Verfahren und Vorrichtungen zum Abfüllen von Füllprodukten bekannt, die ohne Durchflussmesser auskommen. So ist beispielsweise in der DE 10 2014 104 873 A1 eine Technologie zum schlagartigen Befüllen von Behältern beschrieben. Hierbei werden das Füllprodukt unter einem Überdruck bereitgestellt, der zu befüllende Behälter evakuiert und das unter Überdruck stehende Füllprodukt in den unter Unterdruck stehenden Behälter eingeleitet. Aufgrund der so hergestellten Druckdifferenz erfolgt das Einleiten des Füllprodukts schlagartig. Um die Beruhigungszeit des Füllprodukts nach der Befüllung im Behälter zu verkürzen und ein Aufschäumen sowie Überschäumen des Füllproduktes zu verhindern, wird der Behälter unter Überdruck verschlossen, ohne dass zuvor ein Druckausgleich des Behälterinnenraums mit der äußeren Umgebung stattfindet.

Das vorstehend beschriebene Füll-/Verschließsystem benötigt keine Durchflussmesser an den einzelnen Füllstationen, da die Befüllung ausschließlich durch das Vakuum-Druckniveau im evakuierten Behälter und den Fülldruck regelbar ist.

### Darstellung der Erfindung

Ausgehend von dem bekannten Stand der Technik ist es eine Aufgabe der vorliegenden Erfindung, die Validierung des Reinigungs- und/oder Sterilisationsprozesses einer Vorrichtung zum Befüllen eines Behälters mit einem Füllprodukt, vorzugsweise in einer Getränkeabfüllanlage, weiter zu verbessern, insbesondere baulich und/oder verfahrenstechnisch zu vereinfachen.

Die Aufgabe wird durch eine Vorrichtung mit den Merkmalen des Anspruchs 1 sowie einem Verfahren mit den Merkmalen des unabhängigen Verfahrensanspruchs gelöst. Vorteilhafte Weiterbildungen ergeben sich aus den Unteransprüchen, der folgenden Darstellung der Erfindung sowie der Beschreibung bevorzugter Ausführungsbeispiele.

Die erfindungsgemäße Vorrichtung dient zum Befüllen eines Behälters mit einem Füllprodukt. Die Vorrichtung kommt besonders bevorzugt in einer Getränkeabfüllanlage zum Abfüllen von Getränken, wie beispielsweise Wasser, karbonisiert oder nicht-karbonisiert, Softdrinks, Bier oder Mischgetränken, zur Anwendung.

Wenn Bezeichnungen wie "Behälter", "Verschluss" und andere hierin in der Einzahl verwendet werden, geschieht dies in erster Linie der sprachlichen Einfachheit halber. Der Plural ist mitumfasst, sofern dieser nicht ausdrücklich ausgeschlossen ist.

Die Vorrichtung weist zumindest eine Füllstation mit einem Füllorgan zum Einleiten des Füllprodukts in den zu befüllenden Behälter sowie eine Behandlungsvorrichtung zum Behandeln zumindest eines Teils der Füllstation, insbesondere des Füllorgans, mit einem Behandlungsmedium, das sich vom Füllprodukt unterscheidet, auf. Die Behandlungsvorrichtung dient in erster Linie der Reinigung und/oder Sterilisation der Vorrichtung, insbesondere der Füllorgane. Entsprechend wird hierin zwischen einem "regulären Betrieb" der Vorrichtung, in dem eine Abfüllung der Behälter stattfindet, und einem "Behandlungsbetrieb", in dem die Reinigung und/oder Sterilisation stattfindet, unterschieden.

Die Vorrichtung weist gemäß der Erfindung ferner zumindest einen Temperatursensor zum Erfassen einer Temperatur zumindest eines Abschnitts der Füllstation und eine Kontrolleinrichtung auf, die eingerichtet ist, um einen Referenzwert, der einer Temperatur des Behandlungsmediums an einer von der zumindest einen Füllstation abweichenden Stelle entspricht, zu ermitteln, während der Behandlung des Füllorgans die vom Temperatursensor erfasste Temperatur mit dem Referenzwert zu vergleichen und daraus zu ermitteln, ob eine ausreichende Behandlung des Füllorgans vorliegt.

Mit der Formulierung "vom Temperatursensor erfasste Temperatur" sind neben Temperaturwerten in üblichen physikalischen Temperatureinheiten ebenso Parameter mitumfasst, die aus der detektierten Temperatur der betreffenden Füllstation abgeleitet werden und somit ein alternatives Maß für die detektierte Temperatur sind. So kann durch die Kontrolleinrichtung beispielsweise eine Normierung oder anderweitige Umrechnung oder Bearbeitung der empfangenen Messdaten erfolgen, um vom konkreten Aufbau des Temperatursensors, von konkreten physikalischen Einheiten und dergleichen zu abstrahieren. Dies gilt gleichermaßen für den Referenzwert und dessen Ermittlung beziehungsweise Erfassung.

Durch einen Vergleich der gemessenen Füllstationstemperatur, insbesondere der Temperatur des Füllorgans, mit einem Referenzwert während der Reinigung und/oder Sterilisation besteht die Möglichkeit, auch ohne Durchflussmessung des Behandlungsmediums die Reinigung beziehungsweise Sterilisation zu verifizieren. Ferner ist es nicht erforderlich, die Bestimmung, ob die Behandlung ausreichend oder unzureichend ist, anhand der zeitlichen Entwicklung der erfassten Temperatur und durch Anwendung vorgegebener und voreingestellter Schwellwerte durchzuführen. Vielmehr werden während der Behandlung Temperaturwerte an unterschiedlichen Stellen der Vorrichtung miteinander verglichen, wodurch die Verifikation beziehungsweise Überwachung im Wesentlichen allein aus dem Ist-Zustand des Systems durchführbar ist. Sie erfolgt somit auf baulich und verfahrenstechnisch besonders einfache, autarke und zuverlässige Weise.

Das Behandlungsmedium für die Reinigung und/oder Sterilisierung ist oder umfasst vorzugsweise Dampf, besonders bevorzugt Heißdampf. Alternativ oder zusätzlich kann das Behandlungsmedium auch Wasserstoffperoxid oder Peressigsäure, bevorzugt in Kombination mit Alkoholen, etwa Ethanol, aufweisen.

Vorzugsweise weist die Vorrichtung einen Produktkessel in Fluidverbindung mit dem Füllorgan und einen Referenztemperatursensor auf, der zur Bestimmung der Temperatur des im Produktkessel befindlichen Mediums eingerichtet ist. Der Produktkessel fungiert im regulären Betrieb als Füllproduktreservoir und im Behandlungsbetrieb, d.h. Reinigungs- beziehungsweise Sterilisationsbetrieb, als Reservoir für ein Behandlungsmedium, etwa in Form von Heißdampf oder einer Reinigungsflüssigkeit. Die Kontrolleinrichtung ist nun vorzugsweise eingerichtet, um die vom Referenztemperatursensor erfasste Temperatur als Referenzwert heranzuziehen oder daraus abzuleiten.

Auf diese Weise kann der Referenzwert besonders einfach und zuverlässig unmittelbar aus der Bereitstellung beziehungsweise Quelle des Behandlungsmediums gewonnen werden. Entspricht die Temperatur des überwachten Füllorgans in etwa dem Referenzwert oder weicht nur leicht davon ab, liegt diese beispielsweise innerhalb eines zulässigen Bereichs unterhalb der Kesseltemperatur, kann davon ausgegangen werden, dass das Füllorgan ausreichend mit Behandlungsmedium versorgt wird und die Behandlung erfolgreich verläuft. Andernfalls wird das betreffende Füllorgan womöglich nicht ausreichend mit Behandlungsmedium versorgt, und es können entsprechend Gegenmaßnahmen ergriffen werden. So ist es beispielsweise möglich, im letzteren Fall eine Fehlermeldung auszugeben, das Füllorgan zum Auslassen von Kondensat und/oder zur Erhöhung des Durchsatzes mit Behandlungsmedium zu öffnen oder mehr Behandlungsmedium an das entsprechende Füllorgan zu leiten.

Vorzugsweise sind mehrere Füllstationen mit jeweils einem Füllorgan zum Einleiten des Füllprodukts in zu befüllende Behälter vorgesehen. Die Kontrolleinrichtung ist in diesem Fall vorzugsweise eingerichtet, um den Referenzwert aus der Temperatur zumindest einer ersten Füllstation heranzuziehen oder daraus abzuleiten, die vom Temperatursensor erfasste Temperatur einer zweiten Füllstation mit dem Referenzwert zu vergleichen und daraus zu ermitteln, ob eine ausreichende Behandlung des zweiten Füllorgans vorliegt. Es sei darauf hingewiesen, dass die Bezeichnungen "erstes" und "zweites" nur der sprachlichen Unterscheidung zweier zu vergleichender Füllstationen dienen; eine besondere Ordnung oder Reihenfolge ist damit nicht impliziert.

In anderen Worten, gemäß dieser Ausführungsform werden die Temperaturen unterschiedlicher Füllstationen miteinander verglichen, um die Behandlung der Kavitäten auf Plausibilität zu prüfen. Entspricht die Temperatur des überwachten Füllorgans im Wesentlichen den Temperaturen eines, mehrerer oder aller anderen Füllorgane, kann davon ausgegangen werden, dass die Behandlung erfolgreich verläuft. Andernfalls wird das betreffende Füllorgan womöglich nicht ausreichend mit Behandlungsmedium versorgt, und es können entsprechend Gegenmaßnahmen ergriffen werden, wie etwa eine oder mehrere der vorstehend genannten. In diesem Fall kann die Behandlung allein aus einem Vergleich der Füllstationen untereinander verifiziert werden, wodurch sich der Aufbau der Vorrichtung noch weiter vereinfacht. Um ein genaueres Ergebnis zu erhalten, kann diese Maßnahme selbstverständlich mit anderen Maßnahmen, wie etwa der vorstehend beschriebenen Detektion und Verarbeitung der Kesseltemperatur, kombiniert werden.

Vorzugsweise ist der zumindest eine Temperatursensor als berührungsloser Temperatursensor, etwa in Form eines Pyrometers und/oder einer Infrarotkamera, ausgebildet. Dies gilt gleichermaßen für einen etwaigen Referenztemperatursensor.

Wenn ein berührungsloser Temperatursensor zum berührungslosen Erfassen der Temperatur vorgesehen ist, kann die Zuverlässigkeit und Wartbarkeit der Vorrichtung weiter erhöht werden, da ein berührungsloser Sensor außerhalb der Füllstation angeordnet werden kann und damit einfacher zugänglich ist. Zudem kann der Reinigungsaufwand vermindert werden, da insgesamt weniger Bauteile gereinigt werden müssen und insbesondere eine Reinigung von sich relativ zueinander bewegenden Teilen und deren Dichtungen entfällt. Durch die berührungslose Temperaturmessung wird darüber hinaus auch eine Kontaminierung des Füllorgans und damit der füllproduktführenden Wege der Vorrichtung durch einen berührenden Temperatursensor vermieden. Entsprechend kann die Vorrichtung insgesamt kompakter und hygienischer aufgebaut werden. Des Weiteren ist hierdurch eine höhere Prozesssicherheit gegeben, da es bei berührungslos messenden Sensoren nicht wie bei berührend messenden Sensoren zu einem Ablösen des Sensors von der zu messenden Fläche oder zu einem Abrieb oder einer Deformation des Sensors kommen kann.

Die berührungslose Messung der Temperatur kann bevorzugt an einer Außenseite des Füllorgans, bevorzugt im Bereich eines Ventilsitzes des Füllorgans, durchgeführt werden, so dass die Temperatur des Füllorgans berührungslos direkt erfasst und überwacht werden kann. Dabei kann davon ausgegangen werden, dass bei einer Durchleitung beispielsweise von Heißdampf durch die füllproduktberührten Wege bei einer Temperaturmessung am Füllorgan auch die stromaufwärts liegenden Wege zumindest die gleiche Temperatur aufweisen wie das Füllorgan.

Der berührungslose Temperatursensor ist bevorzugt auf eine Außenseite beziehungsweise Außenfläche des entsprechenden Füllorgans gerichtet. Bevorzugt erfasst der berührungslose Temperatursensor die Temperatur des Füllorgans dabei im Bereich des Ventilsitzes. So kann die Temperatur des Füllorgans einfach und präzise erfasst werden.

Insbesondere, wenn der berührungslose Temperatursensor als Infrarotkamera ausgebildet ist, kann dieser die Temperatur mehrerer Füllorgane gleichzeitig erfassen, so dass ein nochmals vereinfachter Aufbau der Vorrichtung und eine nochmals erhöhte Prozesssicherheit erzielt werden. Durch eine entsprechende Auswertungssoftware kann damit für jedes individuelle Füllorgan die Temperatur ermittelt werden.

Vorzugsweise umfasst der zumindest eine Temperatursensor eine elektronische Einrichtung, etwa einen RFID-Chip, zur drahtlosen Übermittlung der gemessenen Temperaturdaten an die Kontrolleinrichtung. Auf diese Weise kann auf eine kabelgebundene Datenübermittlung verzichtet werden, wodurch sich der Aufbau der Vorrichtung weiter vereinfacht und die Flexibilität derselben weiter erhöht werden kann. Der Sensor kann so auch an schwer zugänglichen Stellen verbaut werden, wodurch er besonders bevorzugt für den nachstehend beschriebenen Füller/Verschließer anwendbar ist.

Vorzugsweise ist eine geringere Anzahl an Temperatursensoren als Füllstationen vorgesehen, bevorzugt ist nur ein einziger Temperatursensor zur Erfassung der Temperaturen aller zu überwachender Füllstationen vorgesehen, wobei die entsprechenden Füllorgane relativ zu den Temperatursensoren bewegbar sind.

Wenn eine geringere Anzahl an Temperatursensoren als Füllstationen vorgesehen ist, kann der Aufwand für die Temperaturmessung reduziert werden. Dies ist besonders augenfällig bei der Verwendung nur eines einzigen Temperatursensors für die gesamte Vorrichtung. Da die Füllventile an dem Temperatursensor vorbeibewegt werden, kann dennoch eine zuverlässige Messung der Temperatur aller Füllventile vorgenommen werden. Der Temperatursensor ist dabei bevorzugt an einer festen Position angeordnet, so dass er auch mit der Kontrolleinrichtung besonders einfach verbunden werden kann. So ist die Übergabe des jeweiligen Signals von einem etwaigen drehenden Teil an ein stehendes Teil nicht notwendig. Zudem ist es insbesondere bei Vorrichtungen in Rundläuferbauweise möglich, eine Vielzahl von an dem Rundläuferkarussell angeordneten Füllstationen zu überwachen, wobei die Temperatur der einzelnen Füllstationen jeweils ermittelt werden kann, wenn die jeweilige Füllstation an dem Temperatursensor vorbeibewegt beziehungsweise durch dessen Messbereich bewegt wird. Die Vorrichtung kann dann besonders einfach aufgebaut sein. Zudem ist hierdurch die Störanfälligkeit sowie die Komplexität derselben reduziert, da lediglich ein Sensor vorzusehen ist, mit dem die Temperatur einer Vielzahl von Füllorganen ermittelt werden kann.

Aus den vorstehend genannten Gründen ist die Temperatur der jeweiligen Füllstationen vorzugsweise bei einem Vorbeibewegen derselben an dem zumindest einen Temperatursensor erfassbar, wobei die Füllstationen an einer Transportvorrichtung, bevorzugt einem Rundläuferkarussell, angeordnet sind.

Alternativ können eine Mehrzahl von Füllstationen und eine Mehrzahl von Temperatursensoren vorgesehen sein, wobei jeder Füllstation jeweils (zumindest) ein Temperatursensor zugeordnet ist. Dadurch kann die Temperatur jeder Füllstation, insbesondere jedes Füllorgans, besonders exakt erfasst werden. Zudem ist ein permanentes Erfassen und mithin ein permanentes Überwachen der Temperatur(en) ermöglicht, so dass der Reinigungs- und/oder Sterilisierungsvorgang besonders sicher erfolgen kann. Dadurch kann auch sichergestellt werden, dass die Temperatur einer jeden Füllstation zu jederzeit erfassbar ist und somit ein besonders präzises Überwachen und Steuern/Regeln des Behandlungsvorgangs erzielbar ist.

Vorzugsweise ist die Vorrichtung zum Befüllen und Verschließen des Behälters eingerichtet, in diesem Fall hierin auch als "Füller/Verschließer" bezeichnet, wobei jede Füllstation in diesem Fall vorzugsweise aufweist: eine Kopfkammer, die eingerichtet ist, um den Behälter zumindest teilweise, vorzugsweise einen Mündungsabschnitt des Behälters, aufzunehmen, und die gegenüber der äußeren Umgebung abdichtbar ist; das Füllorgan, das in der Kopfkammer zum Evakuieren des Behälters sowie Einleiten des Füllprodukts in den evakuierten Behälter eingerichtet ist, wobei das Füllprodukt vorzugsweise unter einem Überdruck steht; und ein Verschließorgan mit einem Verschließerkopf, der eingerichtet ist, um einen Verschluss aufzunehmen und den Behälter in der Kopfkammer und ohne vorherige Entlastung auf Umgebungsdruck mit dem Verschluss zu verschließen, wobei der Verschließerkopf vorzugsweise zwischen einer zurückgezogenen Position und einer Verschließposition bewegbar ist.

Die Bezeichnungen "evakuieren", "Vakuum" und dergleichen implizieren hierin nicht unbedingt das Bestreben, den Unterdruck im Behälter möglichst einem perfekten Vakuum anzunähern. Vielmehr kann das Evakuieren dem Aufbau eines spezifischen Unterdrucks dienen. Die Bezeichnungen "Unterdruck" und "Überdruck" sind hierbei zunächst relativ zueinander zu verstehen. Allerdings liegt der Unterdruck nach der Evakuierung des Behälters vorzugsweise unterhalb des Atmosphärendrucks (=Normaldruck). Der Überdruck des Füllprodukts, unter dem vorzugsweise abgefüllt wird, kann dem Atmosphärendruck entsprechen, liegt jedoch vorzugsweise darüber.

So wird der Behälter vor dem Einleiten des Füllprodukts vorzugsweise auf einen Unterdruck P_{low} mit einem Absolutdruck von 0,5 bis 0,05 bar, bevorzugt 0,3 bis 0,1 bar, besonders bevorzugt von etwa 0,1 bar evakuiert. Vorzugsweise liegt der Überdruck oberhalb des Atmosphärendrucks, etwa bei einem Absolutdruck von 1,1 bar bis 6 bar. Vorzugsweise wird der Behälter so evakuiert, dass bei der Befüllung mit dem Füllprodukt im Wesentlichen kein Gas durch das Füllprodukt verdrängt wird und entsprechend auch kein Gas aus dem Innenraum des Behälters ausströmen muss. Vielmehr kann der gesamte Mündungsquerschnitt des Behälters zum Einleiten des Füllprodukts verwendet werden. Mit anderen Worten, es tritt beim Befüllen nur ein in den Behälter hinein gerichteter Füllproduktstrom, jedoch kein entgegengesetzter Fluidstrom auf.

Auf diese Weise finden ein schlagartiges Befüllen des Behälters sowie ein zeitlich und räumlich integriertes und damit ausgesprochen hygienisches Verschließen desselben statt.

Vorzugsweise weist das Füllorgan einen Mündungsabschnitt auf, wobei das Befüllen in diesem Fall vorzugsweise ferner so ausgeführt wird, dass der Mündungsabschnitt zum Befüllen des Behälters mit diesem in der Kopfkammer dichtend in Fluidkommunikation gebracht wird, die Kopfkammer zur äußeren Umgebung hin abgedichtet und auf einen Überdruck gebracht wird, und der Mündungsabschnitt nach Beendigung des Füllprozesses vom Behälter gelöst wird, wodurch auf das Füllprodukt im Behälter der Überdruck der Kopfkammer wirkt. Auf diese Weise findet ein schlagartiges Befüllen des Behälters statt, während gleichzeitig ein Überschäumen nach dem Befüllen, insbesondere nach dem Entfernen des Füllorgans von der Behältermündung, unterbunden wird.

Die hierhin dargelegte Verifikation der Reinigung und/oder Sterilisation der Füllorgane ist für Füller/Verschließer, insbesondere zum schlagartigen Befüllen, besonders geeignet, da der komplexe Aufbau der Füllstationen, umfassend Füllorgan und Verschließer, eine herkömmliche Überwachung erschwert. Insbesondere muss nicht auf eine Durchflussmessung des Behandlungsmediums zurückgegriffen werden.

Vorzugsweise ist der Temperatursensor außerhalb der Kopfkammer angeordnet, so dass eine bauliche Modifikation der Füllstation zur Behandlungsüberwachung nicht erforderlich ist.

Alternativ oder zusätzlich kann der Temperatursensor im Verschließerkopf eingebettet sein. So findet die Temperaturmessung unmittelbar in der Kopfkammer, die bei geöffnetem Füllorgan ebenfalls mit Behandlungsmedium durchspült oder beaufschlagt wird, statt, beispielsweise mittels eines oder mehrerer RFID-Sensoren, welche die Temperaturdaten drahtlos an die Kontrolleinrichtung übermitteln. Ein solcher Temperatursensor kann beispielsweise in die Oberfläche des Verschließerkopfs eingebettet sein, wodurch das Füllorgan baulich unverändert bleiben kann.

So misst der Temperatursensor zwar bevorzugt eine Temperatur am Füllorgan, allerdings kann die Bestimmung der Temperatur auch indirekt erfolgen, etwa durch Erfassung der Temperatur am Verschließerkopf. Durch die bauliche Integration des Füllorgans und Verschließorgans im Fall des Füller/Verschließers kann somit der Verschließerkopf des Verschließorgans zur Temperaturermittlung synergetisch genutzt werden, beispielsweise indem ein RFID-Sensor in den Verschließerkopf eingebettet ist.

Die oben genannte Aufgabe wird ferner durch ein Verfahren zum Behandeln, vorzugsweise Reinigen und/oder Sterilisieren, eines Füllorgans zumindest einer Füllstation einer Vorrichtung wie im vorherigen besprochen, zum Einleiten eines Füllprodukts in einen zu befüllenden Behälter, vorzugsweise in einer Getränkeabfüllanlage, gelöst. Das Verfahren weist auf: Beaufschlagen zumindest eines Teils des Füllorgans mit einem Behandlungsmedium, vorzugsweise Heißdampf oder einer temperierten Reinigungsflüssigkeit; Erfassen einer Temperatur zumindest eines Abschnitts der Füllstation mittels eines Temperatursensors; Ermitteln eines Referenzwerts, der einer Temperatur des Behandlungsmediums an einer von der zumindest einen Füllstation abweichenden Stelle entspricht; Vergleichen der vom Temperatursensor erfassten Temperatur mit dem Referenzwert; und Ermitteln aus dem Vergleich, ob eine ausreichende Behandlung des Füllorgans vorliegt.

Die Merkmale, technischen Wirkungen, Vorteile sowie Ausführungsbeispiele, die in Bezug auf die Vorrichtung beschrieben wurden, gelten analog für das Verfahren.

So wird aus den oben genannten Gründen als Referenzwert vorzugsweise die Temperatur des Behandlungsmediums in einem Produktkessel, der mit dem Füllorgan in Fluidverbindung steht, und/oder die Temperatur eines oder mehrerer anderer Füllstationen herangezogen oder daraus abgeleitet.

Ferner wird aus den oben genannten Gründen die Temperatur der zumindest einen Füllstation vorzugsweise berührungslos gemessen, etwa mittels eines Temperatursensors in Form eines Pyrometers und/oder einer Infrarotkamera.

Besonders bevorzugt wird das Verfahren in einem Füller/Verschließer durchgeführt. In diesem Fall kann der Temperatursensor die Temperatur des Verschließerkopfs erfassen, wobei der Verschließerkopf während der Behandlung in die Kopfkammer bewegt wird. Vorzugsweise wird der Verschließerkopf während der Behandlung zwischen einer zurückgezogenen Position und einer Verschließposition hin- und herbewegt, wodurch der Verschließerkopf die Temperatur der Kopfkammer, bewirkt durch das über das Füllorgan eingebrachte Behandlungsmedium, annimmt.

Die Kontrolleinrichtung ist vorzugsweise eingerichtet, um die Behandlung der mindestens einen Füllstation anhand des Vergleichsergebnisses zu steuern beziehungsweise zu regeln. Dadurch kann eine besonders effiziente und ressourcenschonende Behandlung erzielt werden. Wenn die Kontrolleinrichtung ferner derart ausgebildet ist, dass das zu überwachende Füllorgan während des Behandlungsvorgangs geöffnet wird, wenn der Vergleich eine unzureichende Behandlung anzeigt, kann auch Behandlungsmedium eingespart werden. Zum anderen kann durch einen etwaigen Temperaturabfall entstehendes Kondensat ausgelassen, bevorzugt ausgeblasen, und das Innere der Füllstation, insbesondere des Füllorgans, so lange mit Behandlungsmedium bespült beziehungsweise bedämpft werden, bis der Vergleich wieder eine ausreichende Behandlung kennzeichnet.

Besonders bevorzugt erfolgt das Öffnen und Schließen des Füllorgans beziehungsweise dessen Füllventil alternierend. Vorzugsweise wird das Öffnen/Schließen des Füllventils vergleichsabhängig getaktet. So wird folglich nur dann Behandlungsmedium über das geöffnete Füllventil ausgelassen, wenn es zum Aufheizen und/oder Ablassen von Kondensat erforderlich ist.

Weitere Vorteile und Merkmale der vorliegenden Erfindung sind aus der folgenden Beschreibung bevorzugter Ausführungsbeispiele ersichtlich. Die dort beschriebenen Merkmale können alleinstehend oder in Kombination mit einem oder mehreren der oben dargelegten Merkmale umgesetzt werden, insofern sich die Merkmale nicht widersprechen. Die folgende Beschreibung bevorzugter Ausführungsbeispiele erfolgt dabei mit Bezug auf die begleitenden Zeichnungen.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine schematische Schnittansicht einer Füllvorrichtung zum Abfüllen eines Füllprodukts in einen zu befüllenden Behälter;
- Figur 2: eine schematische Detail-Schnittansicht eines Füllorgans einer Füllvorrichtung gemäß einer alternativen Ausführungsform;
- Figur 3: eine schematische Querschnittsansicht von der Seite betrachtet, die einen Ausschnitt einer Vorrichtung zum Befüllen und Verschließen von Behältern gemäß einer weiteren Ausführungsform zeigt; und
- Figur 4: eine schematische Darstellung einer Füllvorrichtung gemäß einem weiteren Ausführungsbeispiel.

### Detaillierte Beschreibung bevorzugter Ausführungsbeispiele

Im Folgenden werden bevorzugte Ausführungsbeispiele anhand der Figuren beschrieben. Dabei sind gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

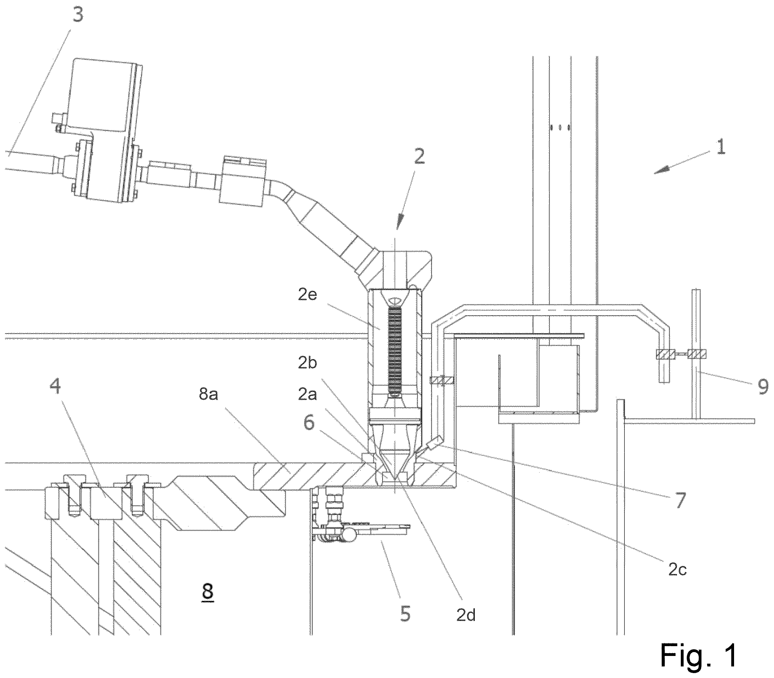

Die Figur 1 ist eine schematische Schnittansicht einer Vorrichtung 1 zum Befüllen eines Behälters mit einem Füllprodukt, hierin auch als "Füllvorrichtung" bezeichnet, in einer Füllproduktabfüllanlage, vorzugsweise Getränkeabfüllanlage. Die Füllvorrichtung 1 ist in Rundläuferbauweise ausgebildet und weist ein drehbares Rundläuferkarussell 4 und einen gegenüber dem Rundläuferkarussell 4 statischen Teil 9 auf.

Ferner ist gemäß dem Ausführungsbeispiel ein Reinraum 8 vorgesehen, in dem das Abfüllen des Füllprodukts in den Behälter in einer kontrollierten Atmosphäre erfolgt. Der Reinraum 8 ist durch einen am Rundläuferkarussell 4 ausgebildeten Reinraumdeckel 8a und in den Figuren nicht weiter gezeigte Reinraumwände ausgebildet.

Am Umfang des Rundläuferkarussells 4 ist eine Vielzahl von Füllstationen mit jeweils einem Füllorgan 2 angeordnet, um in der Füllproduktabfüllanlage einen kontinuierlichen Strom an befüllten Behältern auf dem Rundläuferkarussell 4 produzieren zu können.

Die Füllorgane 2 durchstoßen den Reinraumdeckel 8a derart, dass lediglich deren Produktauslass 2d und das Füllventilbodenstück 6 in den Reinraum 8 münden. Das Füllventilbodenstück 6 kann alternativ als Zentrierglocke ausgeführt sein, falls eine Befüllung von Behältern im angepressten Zustand durchgeführt wird.

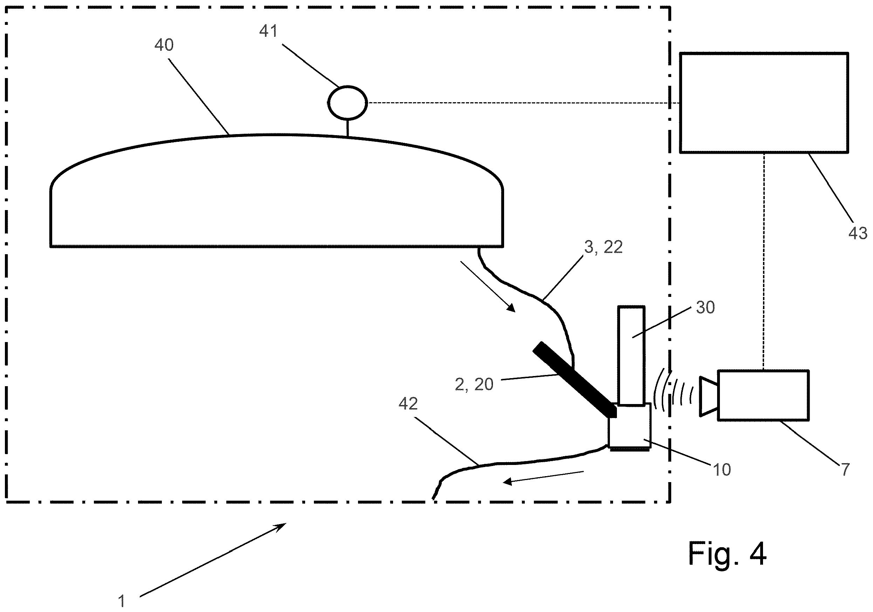

Unterhalb der Füllorgane 2 ist eine Vielzahl von Behälteraufnahmen 5 zum Aufnehmen der zu befüllenden Behälter angeordnet, wobei jeweils eine Behälteraufnahme 5 jeweils einem Füllorgan 2 zugeordnet ist. Die Füllorgane 2 sind jeweils über eine Produktleitung 3 mit einem nicht gezeigten Medienverteiler verbunden, über welchen sowohl das Füllprodukt aus einem Füllproduktreservoir (vgl. Figur 4) als auch ein Behandlungsmedium, etwa in Form von Heißdampf oder einer Reinigungsflüssigkeit, aus einer Sterilisierungsvorrichtung zuleitbar ist.

Die Füllorgane 2 weisen oberhalb ihres Produktauslasses 2d im Ventilinneren 2e einen Ventilkegel 2b auf, der in Richtung der Längsachse des Füllorgans 2 beweglich angeordnet ist und der zum Schließen des Füllorgans 2 in einen Ventilsitz 2a dichtend absenkbar ist. Zum Öffnen des Füllorgans 2 wird der Ventilkegel 2b aus dem Ventilsitz 2a herausgehoben, so dass sich zwischen Ventilkegel 2b und Ventilsitz 2a ein Ringspalt ergibt, durch welchen hindurch das Füllprodukt oder ein Behandlungsmedium dann zum Produktauslass 2d gelangen und aus diesem austreten kann.

An dem stehenden Teil 9 ist ein Temperatursensor 7 angeordnet, der die Temperatur eines vorbeibewegten Füllorgans 2 berührungslos bestimmen kann. Der berührungslose Temperatursensor 7 ist dabei an einer festen Position der Füllvorrichtung 1 vorgesehen. Er ist dabei derart positioniert, dass er im Bereich des Ventilkegels 2a an einer Außenseite 2c des Füllorgans 2 die Temperatur des Füllorgans 2 erfasst.

Der berührungslose Temperatursensor 7 ist dabei so eingerichtet, dass er die Temperatur jeweils eines der Mehrzahl der Füllorgane 2, wenn sie sich an dem berührungslosen Temperatursensor 7 vorbeibewegen, bestimmen kann. Mit anderen Worten kann der berührungslose Temperatursensor 7 die Temperaturen der einzelnen Füllorgane 2 voneinander differenzieren. Der berührungslose Temperatursensor 7 ist dadurch in der Lage, nacheinander die Temperatur eines jeden Füllorgans 2, das mit dem Rundläuferkarussell 4 an ihm vorbeigeführt wird, zu erfassen.

Im Fall eines stationären Temperatursensors 7 gemäß der Figur 1 dreht das Rundläuferkarussell 4 vorzugsweise mit reduzierter Geschwindigkeit. In Abhängigkeit vom Maschinengrobtakt kann der zu einem Zeitpunkt empfangene Messwert der jeweiligen Füllstation beziehungsweise dem jeweiligen Füllorgan 2 zugeordnet werden.

Der berührungslose Temperatursensor 7 ist ferner mit einer in der Figur 1 nicht gezeigten Kontrolleinrichtung (vgl. Figur 4) und/oder Steuerungs- beziehungsweise Regelungseinrichtung verbunden, mittels welcher die Behandlung, beispielsweise die Reinigung und/oder Sterilisation der Füllorgane 2, gesteuert beziehungsweise geregelt sowie die Temperatur der Füllorgane 2 überwacht werden kann.

Der berührungslose Temperatursensor 7 ist beispielsweise als Infrarotkamera ausgebildet. Alternativ kann er jedoch auch als Pyrometer oder in Form eines anderen berührungslosen Temperatursensors ausgebildet sein.

Zum Reinigen beziehungsweise thermischen Sterilisieren der Füllorgane 2 wird über die Produktleitungen 3 vorzugsweise Dampf zu den Füllorganen 2 geleitet. Um ein hinreichendes Bespülen der Kontaktflächen von Ventilkegel 2b und Ventilsitz 2a zu erzielen, werden die Füllorgane 2 zu Anfang bevorzugt in einer offenen Stellung gehalten. Um den Verbrauch an Sterilisationsdampf möglichst gering zu halten, wird das Füllorgan 2 im Anschluss teilweise oder vollständig geschlossen, so dass der Sterilisationsdampf im Wesentlichen im Ventilinneren 2e vorliegt. Alternativ kann auch eine Blende beziehungsweise Sterilisationskappe vor dem Produktauslass 2d angeordnet werden, um einen definierten Volumenstrom des Sterilisationsmediums zu erreichen.

Fällt der berührungslos erfasste Temperaturwert eines bestimmten Füllorgans 2 unter einen weiter unten beschriebenen Referenzwert, so wird das jeweilige Füllorgan 2 beispielsweise geöffnet, das aufgrund des Temperaturabfalls möglicherweise entstandene Kondensat aus dem Ventilinneren 2e ausgelassen und frischer Heißdampf zum Beheizen des Füllorgans 2 zugeführt.

Als besonders vorteilhaft hat sich dabei herausgestellt, das Füllorgan 2 alternierend zu öffnen und wieder zu schließen, wobei das Füllorgan 2 anhand des erfassten Temperaturwerts, mithin temperaturabhängig, getaktet werden kann. Es wird in diesem Fall folglich nur dann Behandlungsmedium über das geöffnete Füllorgan ausgelassen, wenn es zum Aufheizen des Füllorgans 2 und/oder Ablassen von Kondensat erforderlich ist. Auf diese Weise kann Behandlungsmedium eingespart werden.

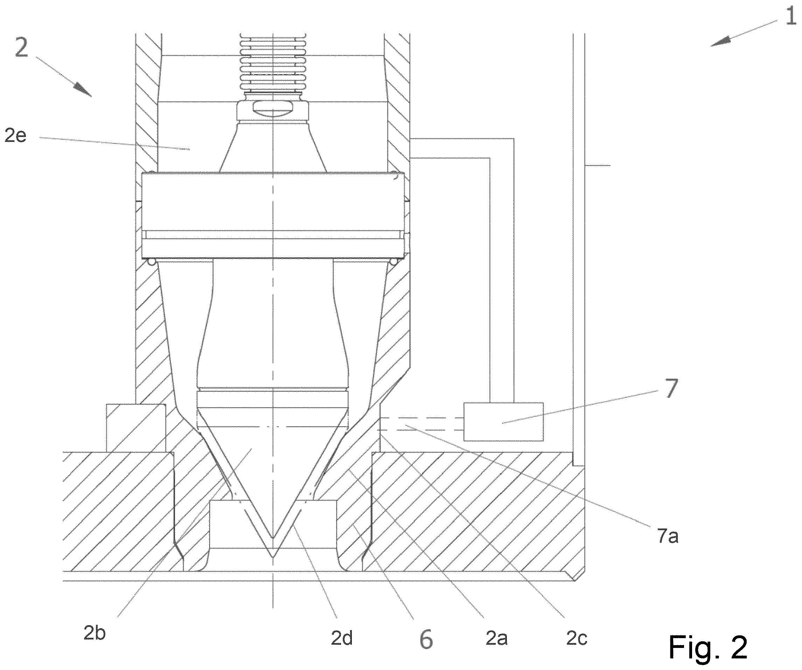

Die Figur 2 zeigt schematisch eine Detail-Schnittansicht eines Füllorgans 2 einer Füllvorrichtung 1 zum Abfüllen eines Füllprodukts in einen zu befüllenden Behälter in einer alternativen Ausführungsform. Die hier gezeigte Füllvorrichtung 1 entspricht im Wesentlichen jener aus der Figur 1. Im Gegensatz zur Füllvorrichtung 1 aus der Figur 1 ist in der in Figur 2 gezeigten Ausführung jeweils ein berührungsloser Temperatursensor 7 an jedem der Mehrzahl von Füllorganen 2 angeordnet. Die Temperatursensoren 7 sind gemäß diesem Ausführungsbeispiel somit am drehenden Teil, also dem Rundläuferkarussell 4, vorgesehen.

In der vorliegenden Ausführungsform sind die berührungslosen Temperatursensoren 7 als Pyrometer ausgebildet. Alternativ können aber auch andere berührungslose Temperatursensoren verwendet werden.

Das Füllorgan 2 ist dabei derart ausgebildet, dass der berührungslose Temperatursensor 7 im Bereich des Ventilsitzes 2a an einer Außenseite 2c des Füllorgans 2 die Temperatur des Füllorgans 2 erfasst. Mit dem Bezugszeichen 7a ist der Messstrahl des Pyrometers angedeutet.

Im Fall der eindeutigen Zuordnung eines Temperatursensors 7 zum Füllorgan 2 gemäß dem Ausführungsbeispiel der Figur 2 kann der Temperatursensor auch durch einen berührend messenden Sensoren realisiert sein, der entweder mit einem Abschnitt des Füllorgans 2 in Kontakt steht und/oder mit dem Behandlungsmedium im Bereich des Füllorgans 2, insbesondere im Bereich des Füllproduktauslasses 2d, in Kontakt kommt.

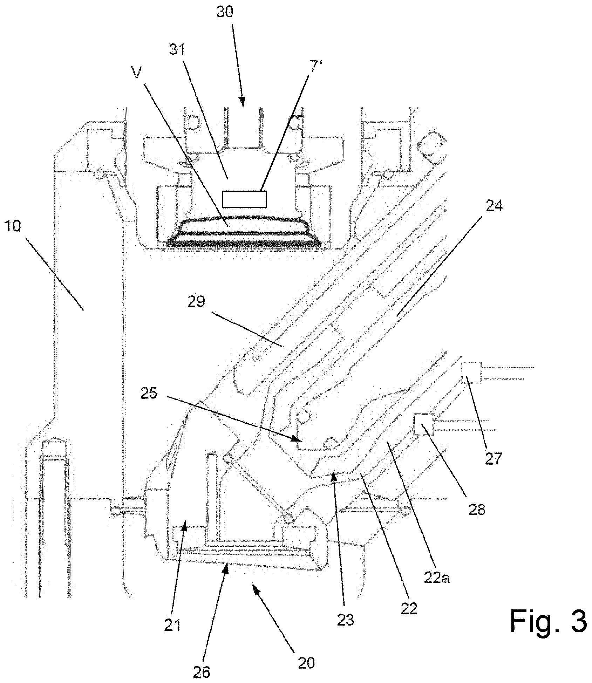

Eine Vorrichtung zum Befüllen und Verschließen von Behältern, hierin auch als "Füller/Verschließer" bezeichnet, gemäß einer weiteren Ausführungsform ist ausschnittsweise in der Figur 3 gezeigt. Die Vorrichtung fällt unter die vorstehend verwendeten Bezeichnungen "Füllvorrichtung" beziehungsweise "Vorrichtung zum Befüllen eines Behälters mit einem Füllprodukt".

Der Füller/Verschließer weist ein Füllorgan 20 auf, das in dem in der Figur 3 gezeigten Prozessstadium in eine Kopfkammer 10 ragt. Das Füllorgan 20 weist aufgenommen in einem Füllorgangehäuse 21 auf: eine Füllproduktleitung 22; ein Füllventil 23, das am unteren, d.h. stromabwärts gelegenen Ende der Füllproduktleitung 22 angeordnet ist; eine Gasleitung 24; und ein Gasventil 25, das am unteren Ende der Gasleitung 24 angeordnet ist.

Über die Gasleitung 24 und das Gasventil 25 kann der Behälter mit einem Gas, etwa Inertgas, Stickstoff und/oder Kohlenstoffdioxid, gespült und/oder vorgespannt werden. Ferner kann der Behälterinnenraum darüber auf einen gewünschten Druck eingestellt, insbesondere evakuiert, werden. Es sei darauf hingewiesen, dass die Gasleitung 24 eine Mehrkanalkonstruktion sein kann, beispielsweise durch einen Rohr-in-Rohr-Aufbau mehrere Gasleitungen umfassen kann, um die Zufuhr von einem oder mehreren Gasen in den Behälter und/oder die Ableitung von Gas aus dem Behälter physisch zu trennen, sofern erforderlich.

Das Gasventil 25 umfasst beispielsweise einen Gasventilkegel und einen Gasventilsitz, die eingerichtet sind, um den Gasdurchfluss zu regeln. Zu diesem Zweck ist der Gasventilkegel über einen nicht dargestellten Aktuator schaltbar.

Die Füllproduktleitung 22 ist vorzugsweise als Ringleitung ausgeführt, die sich im Wesentlichen konzentrisch zur Gasleitung 24 erstreckt. Das Füllventil 23 umfasst beispielsweise einen Füllventilkegel und einen Füllventilsitz, die eingerichtet sind, um den Durchfluss des Füllprodukts zu regeln. Das Füllventil 23 ist eingerichtet, um ein vollständiges Absperren des Füllproduktstroms zu ermöglichen. Im einfachsten Fall weist das Füllventil 23 zwei Stellungen auf, eine geöffnete und eine vollständig geschlossene. Zu diesem Zweck ist das Füllventil 23 über einen nicht dargestellten Aktuator schaltbar.

Die Betätigung des Gasventils 25 und des Füllventils 23 findet über nicht näher dargelegte Aktuatoren statt. Es sei darauf hingewiesen, dass das Gasventil 25 und Füllventil 23 miteinander in Wirkverbindung stehen können, so dass beispielsweise ein Aktuator zur gemeinsamen Nutzung eingerichtet sein kann, um den Aufbau des Füllorgans 20 zu vereinfachen und die Zuverlässigkeit zu erhöhen.

Das Füllorgan 20 weist am Austrittsende der Medien einen Mündungsabschnitt 26 auf, der so eingerichtet ist, dass die Behältermündung dichtend gegen den Mündungsabschnitt 26 gebracht werden kann. Zu diesem Zweck weist der Mündungsabschnitt 26 vorzugsweise eine Zentrierglocke mit einem geeignet geformten Anpressgummi auf. Das Füllorgan 20 mit dem Mündungsabschnitt 26 ist für eine sogenannte Wandfüllung eingerichtet, bei der das Füllprodukt nach Austritt aus dem Mündungsabschnitt 26 an der Behälterwand abwärts strömt. Vorzugsweise sind die Füllproduktleitung 22 und der Mündungsabschnitt 26 so beschaffen oder weisen entsprechende Mittel auf, dass das Füllprodukt beim Abfüllen in Drall versetzt wird, wodurch das Füllprodukt zentrifugalkraftbedingt nach außen getrieben wird und nach Austritt aus dem Mündungsabschnitt 26 in einer Spiralbewegung abwärts strömt.

Optional kann das Füllorgan 20 ein oder mehrere Dosageventile 27, 28 aufweisen, die in einen Dosierraum 22a münden, wodurch ein rascher Sortenwechsel, im Wesentlichen ohne Umstellzeit realisierbar ist.

Die Dosageventile 27, 28 sind bevorzugte Ausprägungen beziehungsweise Ausführungen von Dosagezuleitungen. In anderen Worten: In bestimmten Ausführungsformen, in denen die Einleitung und etwaige Abmessung der Dosagekomponente(n) in den Dosierraum 22a durch bezüglich des Füllorgans 20 externe Mittel realisiert wird, kann gegebenenfalls auf die Dosageventile 27, 28 verzichtet werden, so dass beispielsweise lediglich entsprechende Dosageleitungen oder -kanäle in den Dosierraum 22a münden.

Der Dosierraum 22a kann ein Abschnitt oder geeignet ausgeformter Teil der Füllproduktleitung 22 sein. Über die Dosageventile 27, 28, an welche entsprechende Dosageleitungen angebunden sind, können einer über die Füllproduktleitung 22 in den Dosierraum 22a eingeleiteten Hauptkomponente, beispielsweise Wasser oder Bier, eine oder mehrere Dosagekomponenten, beispielsweise Sirup, Pulpe, Aromen usw., hinzudosiert werden.

Sind Dosageventile 27, 28 vorhanden, werden diese und deren Zuleitungen im weiter unten beschriebenen Behandlungsbetrieb vorzugsweise ebenfalls mit Behandlungsmedium durchspült.

Das Füllorgan 20 ist zumindest teilweise verfahrbar eingerichtet, so dass der in der Figur 3 gezeigte armartige Abschnitt des Füllorgans 20 in die Kopfkammer 10 eingefahren und entweder darin zurückgezogen oder teilweise oder sogar vollständig daraus entfernt werden kann. Dadurch ist es möglich, die Behältermündung für den Abfüllvorgang an den Mündungsabschnitt 26 des Füllorgans 20 anzupressen und anschließend nach Beendigung des Abfüllprozesses das Füllorgan 20 soweit zurückzuziehen, dass der Behälter in der Kopfkammer 10 verschließbar ist.

Um die Verfahrbarkeit des Füllorgans 20 zu gewährleisten, ohne dass die Atmosphäre der Kopfkammer 10 unkontrollierten äußeren Einflüssen ausgesetzt ist, sind entsprechend Mittel, wie etwa aufblasbare Dichtungen, zur Abdichtung vorgesehen. Alternativ oder zusätzlich kann der Kopfkammerdruck nach Beendigung des Abfüllvorgangs größer sein als der Druck der äußeren Umgebung, wodurch ein Eindringen von Verunreinigungen in die Kopfkammer 10 nahezu ausgeschlossen werden kann. Alternativ oder zusätzlich kann sich die Kopfkammer 10 in einem Reinraum befinden oder einen solchen ausbilden.

Der Füller/Verschließer weist ferner ein Verschließorgan 30 zum Verschließen des Behälters auf. Da neben dem Füllorgan 20 hierin weitere Bestandteile, wie etwa die Kopfkammer 10 und das Verschließorgan 30 räumlich integriert als bauliche Einheit am Rundläuferkarussell (in der Figur 3 nicht gezeigt) vorgesehen sind, wird die Füllstation in diesem Fall durch diese baulich Einheit verwirklicht.

Das Verschließorgan 30 weist einen Verschließerkopf31 auf, der in die Kopfkammer 10 ragt und im vorliegenden Ausführungsbeispiel im Wesentlichen vertikal verfahrbar ist. Wie das Füllorgan 20 ist das Verschließorgan 30 zur Wandung der Kopfkammer 10 hin abgedichtet, um eine Kontamination beziehungsweise unkontrollierte Beeinträchtigung der Atmosphäre im Innern der Kopfkammer 10 durch äußere Einflüsse zu vermeiden.

Das Verschließorgan 30 ist dazu ausgebildet und eingerichtet, um am Verschließerkopf31 einen Verschluss V aufzunehmen und zu halten. Zu diesem Zweck kann der Verschließerkopf31 einen Magneten aufweisen, wodurch auf baulich einfache Weise ein Verschluss V, insbesondere wenn dieser ein metallischer Kronkorken ist, zentriert aufgenommen und zum Verschließen des Behälters auf die Behältermündung abgesetzt werden kann. Alternativ kann der Verschluss V durch geeignete Greif- oder Klemmmittel erfasst, gehalten und auf die Behältermündung aufgebracht werden, so dass das hierin dargelegte Konzept insbesondere auch für Kunststoffverschlüsse, Drehverschlüsse usw. anwendbar ist.

Der Verschließerkopf 31 ist in der Auf-/Abrichtung verfahrbar eingerichtet, wobei dieser im Wesentlichen koaxial zur Behältermündung angeordnet ist, um den Verschluss V zuverlässig auf den Behälter applizieren zu können.

Die Übergabe eines Verschlusses V an den Verschließerkopf 31 kann auf verschiedene Art und Weise erfolgen. Beispielsweise kann pro Füll-/Verschließzyklus in einem Schritt zu Beginn des Zyklus ein Verschluss V etwa von einem Sortierwerk und einer Zuführrinne in die Kopfkammer 10 eingebracht werden. Zu diesem Zweck kann die Kopfkammer 10 Teil des Verschließorgans 30 sein und eine Relativbewegung zu der Verschlusszuführung, etwa der Zuführrinne oder einem Übergabearm, ausführen, wobei der Verschließerkopf 31 einen Verschluss V von der Verschlusszuführung pickt und hält.

Es sei darauf hingewiesen, dass das Verschließen des Behälters auch an anderer Stelle erfolgen kann. Insbesondere im Fall kohlenstoffdioxidhaltiger Füllprodukte findet das Verschließen jedoch vorzugsweise unmittelbar nach dem Befüllen und in der Kopfkammer 10 unter Überdruck statt, wie nachstehend erläutert.

Zum Befüllen des Behälters wird dieser beispielsweise relativ zur Kopfkammer 10 angehoben, etwa mittels einer in der Figur 3 nicht dargestellten Hubeinrichtung, die eine plattenförmige Auflage aufweist, die von unten gegen den Behälter fährt und diesen anhebt. Alternativ kann die Kopfkammer 10 abgesenkt und auf die Behältermündung gefahren werden. In diesem Fall kann die Hubeinrichtung genutzt werden, um den Behälter während des Befüllens und Verschließens zu stabilisieren. Selbstverständlich ist auch eine Kombination beider Vorgehensweisen, d.h. Absenkung der Kopfkammer 10 und Hub des Behälters durch die Hubeinrichtung, möglich.

Auf diese Weise wird nun die Behältermündung in die Kopfkammer 10 eingebracht, und die Kopfkammer 10 wird gegenüber der äußeren Umgebung abgedichtet. Die Behältermündung wird dichtend gegen den Mündungsabschnitt 26 des in Füllposition ausgefahrenen Füllorgans 20 angedrückt. Der Mündungsabschnitt 26 des Füllorgans 20 markiert damit die Endposition des Behälterhubs und/oder der Absenkung der Kopfkammer 10.

Der Verschließerkopf 31 nimmt den Verschluss V auf und fährt in die Kopfkammer 10 ein. Die Abdichtung der Kopfkammer 10 gegenüber der Umgebung und gegenüber dem Behälter beziehungsweise dessen Mündungsbereich kann durch geeignete Mittel, etwa aufblasbare Dichtungen, erfolgen.

Während des Füllvorgangs findet vorzugsweise eine Gaszufuhr in die Kopfkammer 10 statt. Durch eine solche Parallelausführung lässt sich der Gesamtprozess optimieren. Während des Füllprozesses ist die Kopfkammer 10 zu allen Seiten hin abgedichtet, wodurch ein geeigneter Innendruck in der Kopfkammer 10 aufgebaut werden kann. Dieser entspricht bei kohlenstoffdioxidhaltigen Füllprodukten vorzugsweise dem Fülldruck oder Sättigungsdruck des Kohlenstoffdioxids oder liegt darüber, wodurch ein Auf- oder Überschäumen des Füllprodukts nach Beendigung des Füllprozesses wirksam unterbunden wird.

Die Gasversorgung für die Kopfkammer 10 kann mittels eines Kopfkammerventils, in der Figur 3 nicht dargestellt, erfolgen. Alternativ oder zusätzlich kann die Gasversorgung zumindest teilweise im Füllorgan 20 integriert sein. So weist zu diesem Zweck das Füllorgan 20 gemäß dem Ausführungsbeispiel der Figur 3 eine Kopfkammergasleitung 29 auf. Die Kopfkammergasleitung 29, insbesondere deren Auslass in die Kopfkammer 10, kann so eingerichtet sein, dass der austretende Gasstrahl auf die Unterseite des Verschlusses V trifft, wenn sich das Füllorgan 20 in der Füllposition befindet. Auf diese Weise findet gleichzeitig eine Reinigung des Verschlusses V während des Füllvorgangs statt. Als Gas wird vorzugsweise Kohlenstoffdioxid verwendet, jedoch ist auch ein anderes Medium, wie zum Beispiel Sterilluft, anwendbar.

Ist nun der Behälter gefüllt und der Innenraum der Kopfkammer 10 auf den gewünschten Druck gebracht, wird das Füllorgan 20 zurückgezogen, und der Verschließerkopf 31 setzt seine Abwärtsbewegung fort, bis beim Erreichen der Behältermündung diese verschlossen wird.

Ein bevorzugter Prozess zum schlagartigen Befüllen des Behälters mit einem Füllprodukt und Verschließen desselben mit einem Verschluss V umfasst:
i) Evakuieren des Behälters auf einen Unterdruck P_{low};
ii) Einfüllen des Füllprodukts in den Behälter, vorzugsweise unter einem Überdruck;
iii) Erzeugen eines Überdrucks P_{high} in der Kopfkammer 10 sowie gegebenenfalls im Kopfraum des Behälters, um beim Lösen des Füllorgans 20 von der Behältermündung ein auf- und überschäumen des Füllprodukts zu vermeiden;
iv) Aufbringen des Verschlusses V auf die Behältermündung und Verschließen des Behälters, ohne vorherige Entlastung auf Umgebungsdruck;
v) Entlüften der Kopfkammer 10 und Ausbringen des Behälters zur weiteren Verarbeitung (bspw. Etikettierung, Verpackung usw.).

Die Bezeichnungen "Unterdruck" und "Überdruck" sind zunächst relativ zueinander zu verstehen. Allerdings liegt der Unterdruck P_{low} nach der Evakuierung im Schritt a) vorzugsweise unterhalb des Atmosphärendrucks (=Normaldruck). Der im Schritt c) erzeugte Überdruck P_{high} kann dem Atmosphärendruck entsprechen, liegt jedoch vorzugsweise darüber.

So wird der Behälter vor dem Einleiten des Füllprodukts vorzugsweise auf einen Unterdruck P_{low} mit einem Absolutdruck von 0,5 bis 0,05 bar, bevorzugt 0,3 bis 0,1 bar, besonders bevorzugt von etwa 0,1 bar evakuiert. Vorzugsweise liegt der Überdruck P_{high} oberhalb des Atmosphärendrucks, etwa bei einem Absolutdruck von 1,1 bar bis 6 bar. Auf diese Weise ist der Behälter so evakuiert, dass bei der Befüllung mit dem Füllprodukt im Wesentlichen kein Gas durch das Füllprodukt verdrängt wird und entsprechend auch kein Gas aus dem Innenraum des Behälters ausströmen muss. Vielmehr kann der gesamte Mündungsquerschnitt des Behälters zum Einleiten des Füllprodukts verwendet werden. Mit anderen Worten, es tritt beim Befüllen nur ein in den Behälter hinein gerichteter Füllproduktstrom, jedoch kein entgegengesetzter Fluidstrom, auf, wodurch der Befüllung weiter beschleunigt werden kann.

Die nachstehend erläuterte Temperaturmessung im Behandlungsbetrieb, d.h. Reinigungs- und/oder Sterilisationsbetrieb, kann mittels eines stationären oder beweglichen Temperatursensors 7 (in der Figur 3 nicht gezeigt), beispielsweise einer Infrarotkamera, außerhalb der Kopfammer 10 erfolgen. Auf diese Weise werden die Konstruktion und Hygiene der Kopfkammer 10 sowie des Füllorgans 20 und Verschließorgans 30 nicht beeinträchtigt. Beispielsweise kann mittels eines als IR-Kamera realisierten Temperatursensors 7 die Abstrahlung der Oberfläche des in die Kopfkammer 10 eintauchenden Verschließorgans 30 gemessen werden. Aus diesem Grund misst der Temperatursensor 7 zwar bevorzugt eine Temperatur am Füllorgan 20, allerdings kann die Bestimmung der Temperatur auch indirekt erfolgen, etwa durch Messung der Temperatur am Verschließerkopf 31.

Der Temperatursensor 7 ist daher zumindest eingerichtet, um eine Temperatur an einem geeigneten Abschnitt der betreffenden Füllstation zu detektieren.

Alternativ kann die Temperaturmessung mittels eines Temperatursensors 7' in der Kopfkammer 10 erfolgen, beispielsweise mittels eines oder mehrerer RFID-Sensoren, welche die Temperaturdaten an die nachstehend beschriebene Kontrolleinrichtung übermitteln. Ein solcher Temperatursensor 7` kann beispielsweise in die Oberfläche des Verschließerkopfs 31 eingebettet sein.

Die Figur 4 ist eine schematische Darstellung einer Füllvorrichtung 1 zur Erläuterung des Behandlungsbetriebs, insbesondere CIP- und/oder SIP-Betriebs. Die Füllvorrichtung 1 weist im Detail beispielsweise den Aufbau der vorstehend beschriebenen Ausführungsbeispiele gemäß den Figuren 1 bis 3 auf.

Die Füllorgane 2, 20 sind jeweils über eine Produktleitung 3, 22 mit einem Produktkessel 40, der im regulären Betrieb als Füllproduktreservoir und im Behandlungsbetrieb als Reservoir für Behandlungsmedium, etwa in Form von Heißdampf oder Reinigungsflüssigkeit, fungiert. Der Produktkessel 40 weist einen Referenztemperatursensor 41 auf, der zur Bestimmung der Temperatur des darin befindlichen Mediums eingerichtet ist. Der Referenztemperatursensor 41 kann berührungslos arbeiten oder mit dem Produktkessel 40 oder dem Medium im Produktkessel 40 in Kontakt stehen.

Der Referenztemperatursensor 41 sowie die Temperatursensoren 7 und/oder 7` sind kommunikativ mit einer Kontrolleinrichtung 43, die eine elektronische Einrichtung zur Überwachung und/oder Steuerung des Behandlungsprozesses ist, verbunden. Die Datenübertragung kann kabellos oder kabelgebunden erfolgen.

Im Reinigungs- beziehungsweise Sterilisationsbetrieb läuft das Behandlungsmedium vom Produktkessel 40 über die Produktleitung 3, 22 zum Füllorgan 2, 20. Im Fall des Füller/Verschließers gemäß der Ausführungsform der Figur 3 werden gegebenenfalls die Gasleitung 24 sowie die Kopfkammer 10 ebenso durchspült beziehungsweise mit dem Behandlungsmedium beaufschlagt. Das Behandlungsmedium wird über einen Rücklauf 42, an den beispielsweise ein Unterdruck angelegt ist, abtransportiert. Das abtransportierte Behandlungsmedium kann entsorgt oder für eine vollständige oder teilweise Wiederverwendung aufbereitet werden.

Zur Überwachung beziehungsweise Validierung der Sterilisation und/oder Reinigung wird die Temperatur der Füllorgane 2, 20 mittels eines oder mehrerer der beschriebenen Temperatursensoren 7, 7' gemessen.

Durch Vergleich der so gemessenen Temperatur(en) mit einem Referenzwert, kann die Reinigung beziehungsweise Sterilisation validiert werden. Der Referenzwert entspricht hierbei einer Temperatur des Behandlungsmediums an einer von der zu überwachenden Füllstation abweichenden Stelle.

So entspricht der Referenzwert vorzugsweise der Kesseltemperatur oder wird aus dieser gewonnen. Alternativ oder zusätzlich kann der Referenzwert der gemessenen Temperatur eines oder mehrerer anderer Füllorgane 2, 20 entsprechen oder daraus gewonnen werden. Der Vergleich kann in regelmäßigen zeitlichen Abständen erfolgen, um auf diese Weise Veränderungen der Temperatur am zu überwachenden Füllorgan 2, 20 festzustellen.

Weicht die Temperatur eines Füllorgans 2, 20 stark vom Referenzwert, also beispielsweise der Kesseltemperatur ab, dann kann daraus geschlossen werden, dass nicht genügend Reinigungsmedium über dieses Füllorgan 2, 20 fließt und die Reinigung und/oder Sterilisation somit ungenügend ist.

Im Fall des Füller/Verschließers gemäß der Ausführungsform der Figur 3 kann während der Reinigung und/oder Sterilisation das Verschließorgan 30 zwischen der zurückgezogenen Position und Verschließposition hin- und herfahren, wodurch das Verschließorgan 30 ebenfalls eine Temperaturerhöhung erfährt, die vom eingebetteten Temperatursensor 7' detektierbar ist.

Das Behandlungsmedium weist im Fall einer Laugenreinigung eine Temperatur von beispielsweise 80°C bis 90°C und Fall einer Säurereinigung von beispielsweise 40°C bis 50°C auf, so dass im Behandlungsbetrieb in der Regel eine Temperaturerhöhung am Füllorgan stattfindet.

Die Idee, wie die Reinigung und/oder Sterilisation jeder Füllstation zu verifizieren ist, basiert darauf, die ansteigende Temperatur einer jeden Station, insbesondere eines jeden Füllorgans 2, 20, während der Behandlung zu detektieren und mit der Temperatur im Vorlauf, vorzugsweise der Kesseltemperatur, zu vergleichen. Befindet sich die Temperatur des überwachten Füllorgans 2, 20 innerhalb eines zulässigen Bereichs unterhalb der Kesseltemperatur, kann davon ausgegangen werden, dass die Behandlung erfolgreich verläuft. Befindet sich die Temperatur des überwachten Füllorgans 2, 20 jedoch stark unterhalb der Kesseltemperatur, insbesondere außerhalb des genannten zulässigen Bereichs, wird das betreffende Füllorgan 2, 20 nicht ausreichend mit Behandlungsmedium versorgt.

Zusätzlich oder alternativ dazu kann auch ein Vergleich zwischen den einzelnen Füllorganen 2, 20 herangezogen werden, um die Behandlung der Kavitäten untereinander auf Plausibilität zu prüfen. Entspricht die Temperatur des überwachten Füllorgans 2, 20 im Wesentlichen den Temperaturen eines, mehrerer oder aller anderen Füllorgane - hierbei kann auch ein Mittelwert herangezogen werden - kann davon ausgegangen werden, dass die Behandlung erfolgreich verläuft. Andernfalls wird das betreffende Füllorgan 2, 20 nicht ausreichend mit Behandlungsmedium versorgt.

Wird das betreffende Füllorgan 2, 20 nicht ausreichend mit Behandlungsmedium versorgt, können Gegenmaßnahmen ergriffen werden. So ist es beispielsweise möglich, eine Fehlermeldung auszugeben, das Füllorgan 2, 20 zum Auslassen von Kondensat und/oder zur Erhöhung des Durchsatzes mit Behandlungsmedium zu öffnen oder mehr Behandlungsmedium an das entsprechende Füllorgan zu leiten. Besonders bevorzugt erfolgt das Öffnen und Schließen des Füllorgans beziehungsweise dessen Füllventil 2, 20 alternierend. In diesem Fall kann das Öffnen/Schließen des Füllventils 2, 20 vergleichsabhängig getaktet werden. So wird folglich nur dann Behandlungsmedium über das geöffnete Füllventil 2, 20 ausgelassen, wenn es zum Aufheizen und/oder Ablassen von Kondensat erforderlich ist.

Durch die hierin dargelegte Auswertung der Temperaturdifferenzen während der Reinigung und/oder Sterilisation besteht die Möglichkeit, auch ohne Durchflussmessung des Behandlungsmediums die Reinigung beziehungsweise Sterilisation zu verifizieren. Ferner ist es nicht erforderlich, die Bestimmung, ob die Behandlung ausreichend oder unzureichend ist, anhand der zeitlichen Entwicklung der erfassten Temperatur und durch Anwendung vorgegebener und voreingestellter Schwellwerte durchzuführen. Vielmehr werden während der Behandlung Temperaturwerte an unterschiedlichen Stellen der Vorrichtung 1 miteinander verglichen, wodurch die Verifikation beziehungsweise Überwachung im Wesentlichen allein aus dem Ist-Zustand des Systems durchführbar ist. Sie erfolgt somit auf baulich und verfahrenstechnisch besonders einfache, autarke und zuverlässige Weise. Zudem ist es nicht erforderlich, jedes einzelne Füllorgan 2, 20 mit Temperatursensoren 7, 7' auszustatten.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Vorrichtung zum Befüllen eines Behälters mit einem Füllprodukt
- 2: Füllorgan
- 2a: Ventilsitz
- 2b: Ventilkegel
- 2c: Außenseite
- 2d: Füllproduktauslass
- 2e: Ventilinneres
- 3: Produktleitung
- 4: Rundläuferkarussell
- 5: Behälteraufnahme
- 6: Füllventilbodenstück
- 7: Temperatursensor
- 7`: Temperatursensor
- 7a: Messstrahl
- 8: Reinraum
- 8a: Reinraumdeckel
- 9: Statischer Teil

- 10: Kopfkammer
- 20: Füllorgan
- 21: Füllorgangehäuse
- 22: Füllproduktleitung
- 22a: Dosierraum
- 23: Füllventil
- 24: Gasleitung
- 25: Gasventil
- 26: Mündungsabschnitt
- 27: Dosageventil
- 28: Dosageventil
- 29: Kopfkammergasleitung
- 30: Verschließorgan
- 31: Verschließerkopf

- 40: Produktkessel
- 41: Referenztemperatursensor
- 42: Rücklauf
- 43: Kontrolleinrichtung

- V: Verschluss

## Patentansprüche

1. Vorrichtung (1) zum Befüllen eines Behälters mit einem Füllprodukt, vorzugsweise in einer Getränkeabfüllanlage, wobei die Vorrichtung (1) aufweist:
zumindest eine Füllstation mit einem Füllorgan (2, 20) zum Einleiten des Füllprodukts in den zu befüllenden Behälter;
eine Behandlungsvorrichtung zum Behandeln, vorzugsweise Reinigen und/oder Sterilisieren, zumindest eines Teils des Füllorgans (2, 20) mit einem Behandlungsmedium;
zumindest einen Temperatursensor (7, 7') zum Erfassen einer Temperatur zumindest eines Abschnitts der Füllstation; und
eine Kontrolleinrichtung (43), die eingerichtet ist, um einen Referenzwert, der einer Temperatur des Behandlungsmediums an einer von der zumindest einen Füllstation abweichenden Stelle entspricht, zu ermitteln, während der Behandlung des Füllorgans (2, 20) die vom Temperatursensor (7, 7`) erfasste Temperatur mit dem Referenzwert zu vergleichen und daraus zu ermitteln, ob eine ausreichende Behandlung des Füllorgans (2, 20) vorliegt.

2. Vorrichtung (1) nach Anspruch 1, **dadurch gekennzeichnet, dass** diese ferner einen Produktkessel (40) in Fluidverbindung mit dem Füllorgan (2, 20) und einen Referenztemperatursensor (41) aufweist, der zur Bestimmung der Temperatur des im Produktkessel (40) befindlichen Mediums eingerichtet ist, wobei die Kontrolleinrichtung (43) eingerichtet ist, um die vom Referenztemperatursensor (41) erfasste Temperatur als Referenzwert heranzuziehen oder daraus abzuleiten.

3. Vorrichtung (1) nach Anspruch 1 oder 2, **dadurch gekennzeichnet, dass** mehrere Füllstationen mit jeweils einem Füllorgan (2, 20) zum Einleiten des Füllprodukts in zu befüllende Behälter vorgesehen sind und die Kontrolleinrichtung (43) eingerichtet ist, um den Referenzwert aus der Temperatur zumindest einer ersten Füllstation heranzuziehen oder daraus abzuleiten, die vom Temperatursensor (7, 7') erfasste Temperatur einer zweiten Füllstation mit dem Referenzwert zu vergleichen und daraus zu ermitteln, ob eine ausreichende Behandlung des zweiten Füllorgans (2, 20) vorliegt.

4. Vorrichtung (1) nach einem der vorigen Ansprüche, **dadurch gekennzeichnet, dass** der zumindest eine Temperatursensor (7, 7`) als berührungsloser Temperatursensor (7), vorzugsweise in Form eines Pyrometers und/oder einer Infrarotkamera, ausgebildet ist.

5. Vorrichtung (1) nach einem der vorigen Ansprüche, **dadurch gekennzeichnet, dass** der zumindest eine Temperatursensor (7, 7') eine elektronische Einrichtung, vorzugsweise einen RFID-Chip, zur drahtlosen Übermittlung der gemessenen Temperaturdaten an die Kontrolleinrichtung (43) umfasst.

6. Vorrichtung (1) nach einem der vorigen Ansprüche, **dadurch gekennzeichnet, dass** eine geringere Anzahl an Temperatursensoren (7, 7') als Füllstationen vorgesehen ist, bevorzugt ein einziger Temperatursensor (7) zur Erfassung der Temperaturen aller zu überwachender Füllstationen, wobei die entsprechenden Füllorgane (2, 20) relativ zu den Temperatursensoren (7) bewegbar sind.

7. Vorrichtung (1) nach Anspruch 6, **dadurch gekennzeichnet, dass** die Temperatur der jeweiligen Füllstationen bei einem Vorbeibewegen derselben an dem zumindest einen Temperatursensor (7) erfassbar ist, wobei die Füllstationen an einer Transportvorrichtung, bevorzugt einem Rundläuferkarussell (4), angeordnet sind.

8. Vorrichtung (1) nach einem der Ansprüche 1 bis 5, **dadurch gekennzeichnet, dass** eine Mehrzahl von Füllstationen und eine Mehrzahl von Temperatursensoren (7, 7`) vorgesehen sind, wobei jeder Füllstation jeweils zumindest ein Temperatursensor (7) zugeordnet ist.

9. Vorrichtung (1) nach einem der vorigen Ansprüche, **dadurch gekennzeichnet, dass** diese zum Befüllen und Verschließen des Behälters eingerichtet ist, wobei jede Füllstation aufweist:
eine Kopfkammer (10), die eingerichtet ist, um den Behälter zumindest teilweise, vorzugsweise einen Mündungsabschnitt des Behälters, aufzunehmen, und die gegenüber der äußeren Umgebung abdichtbar ist;
das Füllorgan (20), das in der Kopfkammer (10) zum Evakuieren des Behälters sowie Einleiten des Füllprodukts in den evakuierten Behälter eingerichtet ist, wobei das Füllprodukt vorzugsweise unter einem Überdruck steht; und
ein Verschließorgan (30) mit einem Verschließerkopf (31), der eingerichtet ist, um einen Verschluss aufzunehmen und den Behälter in der Kopfkammer (10) und ohne vorherige Entlastung auf Umgebungsdruck mit dem Verschluss zu verschließen, wobei der Verschließerkopf (31) vorzugsweise zwischen einer zurückgezogenen Position und einer Verschließposition bewegbar ist.

10. Vorrichtung (1) nach Anspruch 9, **dadurch gekennzeichnet, dass** der zumindest eine Temperatursensor (7, 7`) außerhalb der Kopfkammer (10) angeordnet oder im Verschließerkopf (31) eingebettet ist.

11. Vorrichtung (1) nach Anspruch 9 oder 10, **dadurch gekennzeichnet, dass** der zumindest eine Temperatursensor (7, 7`) eingerichtet ist, um die Temperatur des Verschließerkopfs (31) zu erfassen.

12. Verfahren zum Behandeln, vorzugsweise Reinigen und/oder Sterilisieren, eines Füllorgans (2, 20) zumindest einer Füllstation einer Vorrichtung nach einem der Ansprüche 1 bis 11, zum Einleiten eines Füllprodukts in einen zu befüllenden Behälter, vorzugsweise in einer Getränkeabfüllanlage, wobei das Verfahren aufweist:
Beaufschlagen zumindest eines Teils des Füllorgans (2, 20) mit einem Behandlungsmedium, vorzugsweise Heißdampf oder einer temperierten Reinigungsflüssigkeit;
Erfassen einer Temperatur zumindest eines Abschnitts der Füllstation mittels eines Temperatursensors (7, 7`);
Ermitteln eines Referenzwerts, der einer Temperatur des Behandlungsmediums an einer von der zumindest einen Füllstation abweichenden Stelle entspricht, durch die Kontrolleinrichtung (43);
Vergleichen der vom Temperatursensor (7, 7`) erfassten Temperatur mit dem Referenzwert, durch die Kontrolleinrichtung (43); und
Ermitteln aus dem Vergleich, ob eine ausreichende Behandlung des Füllorgans (2, 20) vorliegt.

13. Verfahren nach Anspruch 12, **dadurch gekennzeichnet, dass** als Referenzwert die Temperatur des Behandlungsmediums in einem Produktkessel (40), der mit dem Füllorgan (2, 20) in Fluidverbindung steht, und/oder die Temperatur eines oder mehrerer anderer Füllstationen herangezogen oder daraus abgeleitet wird.

14. Verfahren nach Anspruch 12 oder 13, **dadurch gekennzeichnet, dass** die Temperatur der zumindest einen Füllstation berührungslos gemessen wird, vorzugsweise mittels eines Temperatursensors (7) in Form eines Pyrometers und/oder einer Infrarotkamera.

15. Verfahren nach einem der Ansprüche 12 bis 14, **dadurch gekennzeichnet, dass** dieses mit einer Vorrichtung nach einem der Ansprüche 1 bis 11 durchgeführt wird, wobei das Verfahren vorzugsweise mit einer Vorrichtung gemäß Anspruch 11 durchgeführt wird, der Temperatursensor (7, 7') die Temperatur des Verschließerkopfs (31) erfasst und der Verschließerkopf (31) während der Behandlung in die Kopfkammer (10) bewegt wird, vorzugsweise zwischen einer zurückgezogenen Position und einer Verschließposition hin- und herbewegt wird.

## Claims

1. A device (1) for filling a container with a fill product, preferably in a beverage filling plant, wherein the device (1) comprises:
at least one filling station having a filling member (2, 20) for introducing the fill product into the container to be filled;
a treatment device for treating, preferably cleaning and/or sterilizing, at least part of the filling member (2, 20) with a treatment medium;
at least one temperature sensor (7, 7`) for detecting a temperature of at least one section of the filling station; and
a control apparatus (43) which is configured to determine a reference value which corresponds to a temperature of the treatment medium at a point other than the at least one filling station, to compare the temperature detected by the temperature sensor (7, 7`) with the reference value during the treatment of the filling member (2, 20) and to determine therefrom whether there is sufficient treatment of the filling member (2, 20).

2. The device (1) according to claim 1, **characterized in that** it further comprises a product tank (40) in fluid connection with the filling member (2, 20) and a reference temperature sensor (41) which is configured to ascertain the temperature of the medium in the product tank (40), wherein the control apparatus (43) is configured to use the temperature detected by the reference temperature sensor (41) as the reference value or derive it therefrom.

3. The device (1) according to claim 1 or claim 2, **characterized in that** a plurality of filling stations is provided, each having a filling member (2, 20) for introducing the fill product into containers to be filled, and the control apparatus (43) is configured to use the reference value from the temperature of at least a first filling station or derive it therefrom, to compare the temperature of a second filling station detected by the temperature sensor (7, 7`) with the reference value and to determine therefrom whether there is sufficient treatment of the second filling member (2, 20).

4. The device (1) according to any of the preceding claims, **characterized in that** the at least one temperature sensor (7, 7`) is configured as a contactless temperature sensor (7), preferably in the form of a pyrometer and/or an infrared camera.

5. The device (1) according to any of the preceding claims, **characterized in that** the at least one temperature sensor (7, 7`) comprises an electronic apparatus, preferably an RFID chip, for wirelessly transmitting the measured temperature data to the control apparatus (43).

6. The device (1) according to any of the preceding claims, **characterized in that** a smaller number of temperature sensors (7, 7') are provided than filling stations, preferably a single temperature sensor (7) for detecting the temperatures of all filling stations to be monitored, wherein the corresponding filling members (2, 20) are movable relative to the temperature sensors (7).

7. The device (1) according to claim 6, **characterized in that** the temperature of the respective filling stations can be detected as they move past the at least one temperature sensor (7), wherein the filling stations are arranged on a transport device, preferably a rotary carousel (4).

8. The device (1) according to any of claims 1 to 5, **characterized in that** a plurality of filling stations and a plurality of temperature sensors (7, 7') are provided, wherein each filling station is assigned at least one temperature sensor (7).

9. The device (1) according to any of the preceding claims, **characterized in that** it is configured to fill and close the container, wherein each filling station comprises:
a head chamber (10) which is configured to at least partially receive the container, preferably a mouth section of the container, and which can be sealed off from the external environment;
the filling member (20) which, in the head chamber (10), is configured to evacuate the container and introduce the fill product into the evacuated container, wherein the fill product is preferably under a positive pressure; and
a closing member (30) having a closing head (31), which is configured to receive a closure and to close the container in the head chamber (10) by means of the closure and without prior relief to ambient pressure, wherein the closing head (31) is preferably movable between a retracted position and a closure position.

10. The device (1) according to claim 9, **characterized in that** the at least one temperature sensor (7, 7`) is arranged outside the head chamber (10) or is embedded in the closing head (31).

11. The device (1) according to claim 9 or claim 10, **characterized in that** the at least one temperature sensor (7, 7`) is configured to detect the temperature of the closing head (31).

12. A method for treating, preferably cleaning and/or sterilizing, a filling member (2, 20) of at least one filling station of a device according to any of claims 1 to 11, for introducing a fill product into a container to be filled, preferably in a beverage filling plant, wherein the method comprises:
exposing at least part of the filling member (2, 20) to a treatment medium, preferably hot steam or a temperature-controlled cleaning liquid;
detecting a temperature of at least one section of the filling station by means of a temperature sensor (7, 7`);
determining a reference value corresponding to a temperature of the treatment medium at a point other than the at least one filling station by means of the control apparatus (43);
comparing the temperature detected by the temperature sensor (7, 7`) with the reference value by means of the control apparatus (43);
and
determining from the comparison whether there is sufficient treatment of the filling member (2, 20).

13. The method according to claim 12, **characterized in that** the temperature of the treatment medium in a product tank (40) which is in fluid connection with the filling member (2, 20) and/or the temperature of one or more other filling stations is used as the reference value or is derived therefrom.

14. The method according to claim 12 or 13, **characterized in that** the temperature of the at least one filling station is measured contactlessly, preferably by means of a temperature sensor (7) in the form of a pyrometer and/or an infrared camera.

15. The method according to any of claims 12 to 14, **characterized in that** it is carried out by means of a device according to any of claims 1 to 11, wherein the method is preferably carried out by means of a device according to claim 11, the temperature sensor (7, 7') detects the temperature of the closing head (31) and the closing head (31) is moved into the head chamber (10) during the treatment, preferably moved back and forth between a retracted position and a closure position.

## Revendications

1. Dispositif (1) permettant le remplissage d'un récipient avec un produit de remplissage, de préférence dans une installation de remplissage de boissons, dans lequel le dispositif (1) présente :
au moins un poste de remplissage comportant un organe de remplissage (2, 20) destiné à introduire le produit de remplissage dans le récipient à remplir ;
un dispositif de traitement permettant le traitement, de préférence le nettoyage et/ou la stérilisation, d'au moins une partie de l'organe de remplissage (2, 20) au moyen d'un milieu de traitement ;
au moins un capteur de température (7, 7`) permettant de détecter une température d'au moins une section du poste de remplissage ; et
un appareil de contrôle (43) qui est configuré pour déterminer une valeur de référence correspondant à une température du milieu de traitement à un endroit différent de l'au moins un poste de remplissage, pour comparer, pendant le traitement de l'organe de remplissage (2, 20), la température détectée par le capteur de température (7, 7`) avec la valeur de référence et pour déterminer, sur cette base, si un traitement suffisant de l'organe de remplissage (2, 20) a lieu.

2. Dispositif (1) selon la revendication 1, **caractérisé en ce qu'**il présente en outre une chaudière à produit (40) en liaison fluidique avec l'organe de remplissage (2, 20) et un capteur de température de référence (41) configuré pour définir la température du milieu présent dans la chaudière à produit (40), dans lequel l'appareil de contrôle (43) est configuré pour utiliser la température détectée par le capteur de température de référence (41) comme valeur de référence ou pour en déduire une valeur de référence.

3. Dispositif (1) selon la revendication 1 ou 2, **caractérisé en ce que** plusieurs postes de remplissage comportant respectivement un organe de remplissage (2, 20) permettant d'introduire le produit de remplissage dans des récipients à remplir sont prévus et l'appareil de contrôle (43) est configuré pour utiliser la valeur de référence à partir de la température d'au moins un premier poste de remplissage ou pour en déduire la valeur de référence, pour comparer la température d'un second poste de remplissage, détectée par le capteur de température (7, 7`), avec la valeur de référence et pour déterminer, sur cette base, si un traitement suffisant du second organe de remplissage (2, 20) a lieu.

4. Dispositif (1) selon l'une des revendications précédentes, **caractérisé en ce que** l'au moins un capteur de température (7, 7`) est conçu sous la forme d'un capteur de température (7) sans contact, de préférence sous la forme d'un pyromètre et/ou d'une caméra infrarouge.

5. Dispositif (1) selon l'une des revendications précédentes, **caractérisé en ce que** l'au moins un capteur de température (7, 7') comprend un appareil électronique, de préférence une puce RFID, pour la transmission sans fil des données de température mesurées à l'appareil de contrôle (43).

6. Dispositif (1) selon l'une des revendications précédentes, **caractérisé en ce qu'**un plus petit nombre de capteurs de température (7, 7') que de postes de remplissage est prévu, de préférence un seul capteur de température (7) permettant de détecter les températures de tous les postes de remplissage à surveiller, dans lequel les organes de remplissage (2, 20) correspondants sont mobiles par rapport aux capteurs de température (7).

7. Dispositif (1) selon la revendication 6, **caractérisé en ce que** la température des postes de remplissage respectifs peut être détectée lors d'un passage de ceux-ci devant l'au moins un capteur de température (7), dans lequel les postes de remplissage sont disposés sur un dispositif de transport, de préférence un carrousel rotatif (4).

8. Dispositif (1) selon l'une des revendications 1 à 5, **caractérisé en ce qu'**une pluralité de postes de remplissage et une pluralité de capteurs de température (7, 7`) sont prévues, dans lequel chaque poste de remplissage est respectivement associé à au moins un capteur de température (7).

9. Dispositif (1) selon l'une des revendications précédentes, **caractérisé en ce qu'**il est conçu pour remplir et fermer le récipient, dans lequel chaque poste de remplissage présente :
une chambre de tête (10) qui est conçue pour recevoir au moins partiellement le récipient, de préférence une section d'embouchure du récipient, et qui peut être rendue étanche par rapport à l'environnement extérieur ;
l'organe de remplissage (20) qui est conçu dans la chambre de tête (10) pour mettre sous vide le récipient ainsi que pour introduire le produit de remplissage dans le récipient mis sous vide, dans lequel le produit de remplissage est de préférence soumis à une surpression ; et
un organe de fermeture (30) comportant une tête de fermeture (31) qui est conçue pour recevoir un élément de fermeture et pour fermer le récipient dans la chambre de tête (10) et sans décharge préalable à pression ambiante au moyen de l'élément de fermeture, dans lequel la tête de fermeture (31) est de préférence mobile entre une position rétractée et une position de fermeture.

10. Dispositif (1) selon la revendication 9, **caractérisé en ce que** l'au moins un capteur de température (7, 7`) est disposé à l'extérieur de la chambre de tête (10) ou est intégré dans la tête de fermeture (31).

11. Dispositif (1) selon la revendication 9 ou 10, **caractérisé en ce que** l'au moins un capteur de température (7, 7`) est configuré pour détecter la température de la tête de fermeture (31).

12. Procédé permettant le traitement, de préférence le nettoyage et/ou la stérilisation, d'un organe de remplissage (2, 20) d'au moins un poste de remplissage d'un dispositif selon l'une des revendications 1 à 11 destiné à introduire un produit de remplissage dans un récipient à remplir, de préférence dans une installation de remplissage de boissons, dans lequel le procédé présente :
l'exposition d'au moins une partie de l'organe de remplissage (2, 20) à un milieu de traitement, de préférence de la vapeur chaude ou un liquide de nettoyage tempéré ;
la détection d'une température d'au moins une section du poste de remplissage à l'aide d'un capteur de température (7, 7`) ;
la détermination, par l'appareil de contrôle (43), d'une valeur de référence correspondant à une température du milieu de traitement à un endroit différent de l'au moins un poste de remplissage ;
la comparaison, par l'appareil de contrôle (43), de la température détectée par le capteur de température (7, 7`) avec la valeur de référence ;
et
la détermination, à partir de la comparaison, du fait de savoir si un traitement suffisant de l'organe de remplissage (2, 20) a lieu.

13. Procédé selon la revendication 12, **caractérisé en ce que** la température du milieu de traitement dans une chaudière à produit (40), laquelle chaudière à produit est en liaison fluidique avec l'organe de remplissage (2, 20), et/ou la température d'un ou de plusieurs autres postes de remplissage sont utilisées comme valeur de référence ou pour en déduire la valeur de référence.

14. Procédé selon la revendication 12 ou 13, **caractérisé en ce que** la température de l'au moins un poste de remplissage est mesurée sans contact, de préférence à l'aide d'un capteur de température (7) sous la forme d'un pyromètre et/ou d'une caméra infrarouge.

15. Procédé selon l'une des revendications 12 à 14, **caractérisé en ce qu'il** est mis en oeuvre au moyen d'un dispositif selon l'une des revendications 1 à 11, dans lequel le procédé est de préférence mis en oeuvre au moyen d'un dispositif selon la revendication 11, le capteur de température (7, 7') détecte la température de la tête de fermeture (31), et la tête de fermeture (31) est déplacée dans la chambre de tête (10) pendant le traitement, de préférence en va-et-vient entre une position rétractée et une position de fermeture.
